# EUROPEAN PATENT APPLICATION

(11) **EP 4 729 048 A1**
(43) Date of publication of application: **22.04.2026**
(21) Application number: 25208455.3
(22) Date of filing: 14.10.2025
(51) Int. Cl.: A61K 9/00, A61K 9/20, A61K 31/5513

(54) **ORALLY DISPERSIBLE TABLETS OF OXAZEPAM**

(30) Priority: 15.10.2024 EP 24383133
(71) Applicant: Neuraxpharm Pharmaceuticals, S.L., 08970 Sant Joan Despí Barcelona (ES)
(72) Inventor: ESCACENA CAMPOS, Alejandro, 08970 SANT JOAN DESPÍ (ES); GARCÍA GARCÍA, Adrian, 08970 SANT JOAN DESPÍ (ES); UBEDA PEREZ, Carmen, 08970 SANT JOAN DESPÍ (ES); DÍEZ MARTÍN, Ignacio, 08970 SANT JOAN DESPÍ (ES)
(74) Representative: ZBM Patents - Zea, Barlocci & Markvardsen

(57) **Abstract**

It relates to orally disintegrating tablets, which are useful in the treatment of anxiety, insomnia and in the control of symptoms of alcohol withdrawal syndrome, and which comprise an intragranular and an extragranular phase, comprising: a) oxazepam or their salts, and b) one or more disintegrants in a an amount from 0.5 to 12.0% by weight with respect to the total weight of the orally disintegrating tablet, and selected from the group consisting of sodium croscarmellose, crospovidone, low-substituted hydroxypropyl cellulose and mixtures thereof, the oxazepam or their salts being in the intragranular phase, as well as the process for their preparation.

## Description

### Field of the invention

The present invention relates to an orally dispersible tablet comprising oxazepam.

### Background art

Oxazepam, having the IUPAC chemical name of 7-chloro-3-hydroxy-5-phenyl-1,3-dihydro-1,4-benzodiazepin-2-one, is an active pharmaceutical ingredient developed for the treatment of anxiety, insomnia and the control of symptoms of alcohol withdrawal syndrome.

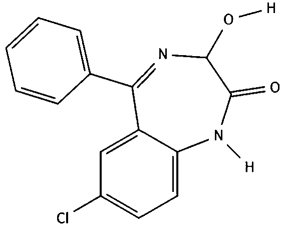

It is sold as tablets under the trademark name Seresta^{®} in strengths of 5 mg, 10 mg, 25 mg and 50 mg. Although tablets are easy to take, they require water which in patients with swallowing problems, especially those suffering from anxiety, can be troublesome.

Orally dispersible tablets provide a good alternative to conventional tablets, as they do not require water to swallow and thus help in patient acceptance and compliance. Moreover, orally dispersible tablets are quickly disintegrated, which allows for a quick absorption, a rapid onset of action and a reduction of drug loss properties.

A standard process for manufacturing orally dispersible tablets usually involves direct compression. However, most active ingredients, including oxazepam, tend to have compressibility problems which leads to tablets having poor flow properties. This has a direct impact on the pharmacokinetic properties of the orally dispersible tablet, such as dissolution profile, a critical quality attribute in order to obtain a bioequivalent product to that already marketed.

Another key critical quality attribute of an orally disintegrating tablet is the so-called disintegration time.

Finally, a major drawback of orally dispersible tablets is their physical and chemical stability in the final package as, due to their requirement of ease of disintegration, orally dispersible tablets tend to lack mechanical strength.

Thus, for what has been mentioned above there remains the need of an orally dispersible tablet comprising oxazepam that can be prepared in a rapid and reproducible manner, possesses proper physicochemical and pharmacotechnical properties in terms of disintegration time, physical stability and dissolution profile, and is bioequivalent to the marketed product Seresta^{®}.

### Summary of the invention

The present invention is directed to an orally dispersible tablet comprising oxazepam. In particular, the present invention is based on the finding that an orally dispersible tablet comprising oxazepam can be prepared in a rapid and reproducible manner when using certain disintegrants in specific amounts. The resulting orally dispersible tablet possesses suitable disintegration time, shows both good physical and chemical stability in the commercial package, has a similar dissolution profile and is bioequivalent to the marketed product Seresta^{®}.

Accordingly, the present invention provides an orally disintegrating tablet comprising an intragranular and an extragranular phase, comprising:
a) oxazepam or a pharmaceutically acceptable salt thereof, and
b) ) one or more disintegrants in an amount from 0.5 to 12.0% by weight with respect to the total weight of the orally disintegrating tablet and selected from the group consisting of croscarmellose sodium, crospovidone, low-substituted hydroxypropyl cellulose and mixtures thereof.

Another aspect of the present invention relates to a process for the preparation of an orally dispersible tablet as defined above comprising: i) providing oxazepam or a pharmaceutically acceptable salt thereof; ii) (a) blending said oxazepam or pharmaceutically acceptable salt thereof with a mixture comprising a diluent, selected from the group consisting of lactose monohydrate, microcrystalline cellulose, cellulose powder and silicified microcrystalline cellulose, and optionally a disintegrant, selected from the group consisting of sodium croscarmellose, crospovidone, low-substituted hydroxypropyl cellulose and mixtures thereof, (b) granulating the resulting blend obtained in step (a); iii) screening the granules resulting from step (ii)(b); iv) blending the screened granules of step (iii) with an extragranular mixture of excipients optionally comprising a disintegrant, and v) compressing the blend resulting from step (vi) to provide a tablet, wherein the amount of disintegrant is in a range from 0.5% to 12.0% by weight with respect to the total weight of the orally dispersible tablet. The orally dispersible tablet may also be defined by its preparation process, thus, the orally dispersible tablet obtainable by the above process is also part of the invention.

Finally, another aspect of the present invention relates to an orally dispersible tablet as defined above, for use in the treatment of anxiety, insomnia and in the control of symptoms of alcohol withdrawal syndrome.

### Brief Description of Drawings

Fig. 1. Shows the dissolution profile of examples 1 and 2 (Ex = example) compared with the dissolution profile of Seresta^{®} 10 mg.
Fig. 2. Shows the dissolution profile of examples 3 and 4 compared with the dissolution profile of Seresta ^{®} 50 mg and with the dissolution profile of comparative example A (comp. ex. A = comparative example A). % oxazepam = percentage of oxazepam dissolved from the orally dispersible tablet. t(s) = time in seconds.

### Detailed description of the invention

As used herein the term orally disintegrating tablet refers to solid dosage forms which disintegrate in the mouth within seconds upon contact with saliva, without the need for additional liquid.

The terms intragranular phase and extragranular phase are synonymous with the terms internal phase and external phase, respectively. The extragranular phase is defined as being anything else of the drug product than the intragranular phase (not taking into account any packaging materials or components). The intragranular phase is defined as that phase that is composed of all the components used for the granulation process and which make up the granules.

The term granulation is used herein to define a process in which primary powder particles are made to adhere to form larger multi-particle entities referred to as granules. Known methods of granulation are dry granulation (utilizing no liquids, e.g. slugging, roller compaction), wet granulation (utilizing liquids such as water or alcohols, e.g. granulation by high-speed mixers, spray-drying, fluidized-bed granulation, extrusion-spheronization- pelletization) and are described in detail by pharmaceutical text books, e.g. Michael E. Aulton, Aulton's Pharmaceutics.

The term granules is used herein as synonymous with granulates and understood as products of the granulation process.

As used herein, the term pharmaceutically acceptable salt refers to a salt prepared from a base or acid which is acceptable for administration to a patient, such as a mammal. Such salts can be derived from pharmaceutically acceptable inorganic or organic bases and from pharmaceutically acceptable inorganic or organic acids.

Pharmaceutically acceptable acids include both inorganic acids such as hydrochloric, sulphuric, phosphoric, diphosphoric, hydrobromic, hydroiodic or nitric acid and organic acids such as citric, fumaric, maleic, malic, ascorbic, succinic, tartaric, benzoic, acetic, methanesulphonic, ethanesulphonic, benzenesulphonic, p-toluenesulphonic acid, formic, acetic, propionic, glycolic, lactic, pyruvic, oxalic, salicylic, trichloroacetic, picric, trifluoroacetic, cinnamic, pamoic, malonic, mandelic, bismethylene salicylic, ethanedisulfonic, gluconic, citraconic, aspartic, stearic, palmitic, EDTA, paminobenzoic or glutamic acid, sulfates, nitrates, phosphates, perchlorates, borates, acetates, benzoates, hydroxynaphthoates, glycerophosphates or ketoglutarates. Further examples of pharmaceutically acceptable inorganic or organic acid salts include the pharmaceutically acceptable salts listed in Journal of Pharmaceutical Science, 66, 2 (1977) which are known to the skilled artisan.

Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines, lysine, guanidine, diethanolamine and choline.

As used herein, the term disintegrant refers to a substance or mixture of substances added to a tablet to facilitate its breakup or disintegration after administration. Suitable disintegrants include but are not limited to croscarmellose sodium, crospovidone, low-susbtituted hydroxypropyl cellulose and mixtures thereof.

As used herein, the term glidant refers to a substance that improves the flow characteristics of a powder mixture. Typical glidants include, but are not limited to talc, calcium silicate, silica and sodium lauryl sulphate.

As used herein, the term lubricant refers to a substance that prevents adhesion of the tablet material to the surface of the dies and punches, reduces interparticle friction, facilitates the ejection of the tablets from the die cavity and improves the rate of flow of the tablet granulation. Typical lubricants include, but are not limited to, calcium stearate, zinc stearate, glyceryl behenate, magnesium stearate and mineral oil.

As used herein, the term binder refers to agents used to impart cohesive qualities to the powdered material so that the tablet remains intact after compression and to improve the free-flowing qualities by the formulation of granules of desired hardness and size. Typical binders include but are not limited to povidone, rice starch, gelatin and polyethylene glycol.

As used herein, the term mixture of extragranular excipients refers to a mixture comprising pharmaceutically acceptable excipients such as a glidant, a binder and a lubricant.

As used herein, the term disintegration time refers to the time the orally disintegrating tablet takes to reach complete disintegration upon contact with salivary fluid.

Orally disintegrating tablets have a disintegration time of not more than 40 seconds. In the tables of the present application, "s" refers to seconds.

As used herein, mean resistance to crushing refers to the force needed to disrupt the tablets by crushing.

As used herein, friability refers to the tendency of the orally dispersible tablets to chip, crumble or break.

As used herein, a dissolution profile is the extent of active pharmaceutical ingredient dissolved in a defined dissolution medium as a function of time from a dosage form, such as an orally dispersible tablet.

As used herein, two medicinal products containing the same active substance are considered bioequivalent if they are pharmaceutically equivalent or pharmaceutical alternatives and their bioavailabilities (rate and extent measured as the plasma concentration time curve) after administration in the same molar dose lie within acceptable predefined limits. These limits are set to ensure comparable in vivo performance, i.e. similarity in terms of safety and efficacy.

As used herein, a fluid bed dryer is a process equipment used to reduce the moisture content of chemical powder and granules.

As mentioned above an orally disintegrating tablet comprising an intragranular and an extragranular phase, comprising: a) oxazepam or a pharmaceutically acceptable salt thereof, and b) one or more disintegrants in an amount from 0.5 to 12% by weight with respect to the total weight of the orally disintegrating tablet, and selected from the group consisting of croscarmellose sodium, crospovidone, low-substituted hydroxypropyl cellulose and mixtures thereof is part of the present invention.

An orally disintegrating tablet according to the first aspect of the present invention is easier to take than a conventional tablet, which improves patient acceptance especially in those with shallowing problems.

In addition, an orally disintegrating tablet according to the first aspect of the present invention shows excellent mechanical properties in terms of physical stability both in a blister package of PVC-Al and in a blister package of Al-Al. In particular, the values are not too low so that the tablets are resistant to crushing and on the other hand, the values are not too high so that the tablets can disintegrate within the specified time limit of not more than 40 seconds.

As used herein, a blister pack is any of several types of pre-formed packaging used for pharmaceutical dosage forms. The primary component of a blister pack is a cavity or pocket made from a formable/extrudable web, usually a thermoformed plastic or extruded aluminium. This usually has a backing of paperboard or a lidding seal of aluminum foil or plastic.

An aluminium-aluminium package, also referred herein to as Al-Al, refers to a package where both the bottom and lid are made of aluminium-based film.

A polyvinyl chloride-aluminium package, also referred herein to as PVC-Al or PVC package, refers to a package where the bottom is made of aluminium and the lid is made of a transparent material such as PVC.

As used herein, the physical stability of the orally dispersible tablets of the present invention is measured in terms of its resistance to crushing and friability.

It is an advantageous finding of the present invention that orally dispersible tablets comprising oxazepam show a similar dissolution profile and are bioequivalent to the marketed 5, 10, 25 and 50 mg strengths of Seresta^{®}.

In a preferred embodiment of the orally disintegrating tablet of the invention, the one or more disintegrants are in the extragranular phase or in both the extragranular and the intragranular phase, preferably in the extragranular phase.

Orally dispersible tablets wherein the one or more disintegrants are in the extragranular phase show disintegration times of less than 30 seconds, preferably in the range of 15 to 22 seconds.

In a preferred embodiment of the orally disintegrating tablet of the invention, the oxazepam or the pharmaceutically acceptable salt thereof is in the intragranular phase.

In a preferred embodiment of the orally disintegrating tablet of the invention, the amount of disintegrant is in a range from 1.0 to 8.0% by weight, more preferably 2.0% to 4%, even more preferably of 3% by weight of the total weight of the orally dispersible tablet. Orally dispersible tablets according to this embodiment show similar dissolution profiles and are bioequivalent to all the strengths of the marketed product Seresta^{®}.

In a more preferred embodiment of the orally disintegrating tablet of the invention, the disintegrant is croscarmellose sodium.

In another more preferred embodiment of the first aspect of the invention, the disintegrant is in an amount in a range from 2.0% to 4.0% by weight with respect to the total weight of the orally dispersible tablet and the disintegrant is croscarmellose sodium.

In an even more preferred embodiment of the first aspect of the invention, the disintegrant is croscarmellose, is found only in the extragranular phase of the orally dispersible tablet and represents from 2.0% to 4.0% by weight with respect to the total weight of the orally dispersible tablet.

In a preferred embodiment of the orally disintegrating tablet of the invention, the amount of oxazepam is in a range from 5.0% and 35.0% by weight with respect to the total weight of the orally disintegrating tablet.

In another embodiment of the first aspect of the present invention, the orally disintegrating tablet comprises an intragranular and an extragranular phase, comprising: a) oxazepam or a pharmaceutically acceptable salt thereof, and b) one or more disintegrants in an amount from 6-10% by weight with respect to the total weight of the orally disintegrating tablet, wherein the one or more disintegrant comprises a mixture of crospovidone and low-substituted hydroxypropyl cellulose.

In another embodiment of the first aspect of the present invention, the orally disintegrating tablet comprises an intragranular and an extragranular phase, comprising: a) oxazepam or a pharmaceutically acceptable salt thereof, and b) one or more disintegrants in an amount from 6-10% by weight with respect to the total weight of the orally disintegrating tablet, wherein the one or more disintegrant is a mixture of crospovidone and low-substituted hydroxypropyl cellulose. In another embodiment of the first aspect, the orally disintegrating tablet further comprises one or more diluents selected from the group consisting of lactose monohydrate, microcrystalline cellulose, cellulose powder and silicified microcrystalline cellulose, preferably lactose monohydrate and microcrystalline cellulose.

In another embodiment of the first aspect, the orally disintegrating tablet further comprises one or more binders selected from the group consisting of povidone, rice starch, gelatine and polyethylene glycol, preferably rice starch.

In another embodiment of the first aspect, the orally disintegrating tablet further comprises one or more glidants, selected from the group consisting of talc, calcium silicate, silica, talc and sodium lauryl sulphate, preferably talc.

In another embodiment of the first aspect, the orally disintegrating tablet further comprises one or more lubricants, selected from the group consisting of calcium stearate, zinc stearate, glyceryl behenate, magnesium stearate and mineral oil, preferably magnesium stearate.

In a more preferred embodiment, the amount of lubricant is in a range from 0.6% to 1% by weight with respect to the total weight of the orally dispersible tablet, preferably of 0.8%.

In another embodiment of the first aspect, the orally disintegrating tablet comprises: a) the oxazepam or a pharmaceutically acceptable salt thereof is in an amount from 7.5% to 9.5% by weight with respect of the total weight of the orally disintegrating tablet, b) the one or more disintegrants are in an amount from 2.0% to 4.0% by weight with respect to the total weight of the orally disintegrating tablet, wherein the disintegrant is in the extragranular phase and the oxazepam is in the intragranular phase of the orally disintegrating tablet.

In another embodiment of the first aspect, the orally disintegrating tablet comprises:
a) oxazepam or a pharmaceutically acceptable salt thereof is in an amount from 7.5% to 9.5% by weight with respect of the total weight of the orally disintegrating tablet,
b) one or more disintegrants in an amount from 2.0% to 4.0% by weight with respect to the total weight of the orally disintegrating tablet, wherein the disintegrant is in the extragranular phase and is croscarmellose, and the oxazepam is in the intragranular phase of the orally disintegrating tablet.

An orally dispersible tablet according to this embodiment shows a comparable dissolution profile to the 10 mg strength of the marketed tablet Seresta^{®} and is bioequivalent to both the 5 and the 10 mg strength.

In another embodiment of the first aspect, the orally disintegrating tablet comprises: a) the oxazepam or a pharmaceutically acceptable salt thereof is in an amount from 20.0% to 30.0% by weight with respect of the total weight of the orally disintegrating tablet, b) the one or more disintegrants are in an amount from 2.0% to 4.0% by weight with respect to the total weight of the orally disintegrating tablet, wherein the disintegrant is in the extragranular phase and the oxazepam is in the intragranular phase of the orally disintegrating tablet.

In another embodiment of the first aspect, the orally disintegrating tablet comprises: a) the oxazepam or a pharmaceutically acceptable salt thereof is in an amount from 20.0% to 30.0% by weight with respect of the total weight of the orally disintegrating tablet, b) the one or more disintegrants are in an amount from 2.0% to 4.0% by weight with respect to the total weight of the orally disintegrating tablet, wherein the disintegrant is in the extragranular phase and is croscarmellose, and the oxazepam is in the intragranular phase of the orally disintegrating tablet.

An orally dispersible tablet according to this embodiment shows a comparable dissolution profile to the 50 mg strength of the marketed tablet Seresta^{®} and is bioequivalent to both the 25 and the 50 mg strength.

In another embodiment of the first aspect, the amount of oxazepam is of 5 mg, 10 mg, 25 mg or 50 mg.

In a preferred embodiment of the orally disintegrating tablet of the invention, the disintegration time of the tablet is between 10 and 30 seconds, preferably between 15 and 22 seconds. These disintegrating times are considered adequate to allow a rapid swallowing of the tablet. The disintegration time is measured according to the standard method described in Ph. Eur. 2.9.1 (Test A). A skilled in the art is well familiar with this procedure.

The orally dispersible tablet as defined in the first aspect comprising can be prepared by a process comprising:
i) providing oxazepam or a pharmaceutically acceptable salt thereof;
ii) (a) blending said oxazepam or pharmaceutically acceptable salt thereof with a mixture comprising a diluent, selected from the group consisting of lactose monohydrate, microcrystalline cellulose, cellulose powder and silicified microcrystalline cellulose, and optionally a disintegrant, selected from the group consisting of sodium croscarmellose, crospovidone, low-substituted hydroxypropyl cellulose and mixtures thereof,
   (b) granulating the resulting blend obtained in step (a)
iii) screening the granules resulting from step (ii)(b);
iv) blending the screened granules of step (iii) with an extragranular mixture optionally comprising a disintegrant, and
v) compressing the blend resulting from step (vi) to provide a tablet,
wherein the amount of disintegrant is in a range from 0.5% to 12.0% by weight with respect to the total weight of the orally dispersible tablet.

Orally dispersible tablets obtained by this method possess a similar dissolution profile to all the strengths with which Seresta^{®} is marketed. On the other hand, orally dispersible tablets comprising oxazepam prepared by the method of direct compression show a dissolution profile significantly slower than that of Seresta^{®} (see comparative example A).

Steps ii)(a) and ii)(b) of the second aspect of the present invention are carried out in a high-shear mixer granulator for a period of 20 to 30 minutes.

In a preferred embodiment of the second aspect of the present invention the blend of step (ii)(a) does not comprise a disintegrant.

In a more preferred embodiment of the second aspect of the present invention, the extragranular excipients comprise a disintegrant.

In a more preferred embodiment of the second aspect of the present invention, the granulation of step (ii)(b) is a wet granulation with an aqueous solution comprising a binder selected from the group consisting of povidone, rice starch, gelatine and polyethylene glycol, preferably rice starch; and has the further step (c) of drying in a fluid bed dryer the granules resulting from step (ii)(b).

In an even more preferred embodiment of the second aspect, the extragranular excipients comprise a lubricant selected from the group consisting of calcium stearate, zinc stearate, glyceryl behenate, magnesium stearate and mineral oil, preferably magnesium stearate.

Another embodiment of the second aspect of the present invention is directed to the preparation of an orally dispersible tablet as defined in the first aspect comprising:
i) providing oxazepam or a pharmaceutically acceptable salt thereof;
ii) (a) blending said oxazepam or pharmaceutically acceptable salt thereof with a mixture comprising a diluent, selected from the group consisting of lactose monohydrate, microcrystalline cellulose, cellulose powder and silicified microcrystalline cellulose,
   (b) granulating the resulting blend obtained in step (a);
iii) screening the granules resulting from step (ii)(b);
iv) blending the screened granules of step (iii) with an extragranular mixture comprising a disintegrant, and
v) compressing the blend resulting from step (vi) to provide a tablet,
wherein the amount of disintegrant is in a range from 2.0% and 4.0% by weight with respect to the total weight of the orally dispersible tablet and the amount of oxazepam is in a range from 7.5% and 9.5% by weight with respect to the total weight of the orally dispersible tablet.

Another embodiment of the second aspect of the present invention is directed to the preparation of an orally dispersible tablet as defined in the first aspect comprising:
i) providing oxazepam or a pharmaceutically acceptable salt thereof;
ii) (a) blending said oxazepam or pharmaceutically acceptable salt thereof with a mixture comprising a diluent, selected from the group consisting of lactose monohydrate, microcrystalline cellulose, cellulose powder and silicified microcrystalline cellulose,
   (b) granulating the resulting blend obtained in step (a);
iii) screening the granules resulting from step (ii)(b);
iv) blending the screened granules of step (iii) with an extragranular mixture comprising a disintegrant, and
v) compressing the blend resulting from step (vi) to provide a tablet,
wherein the amount of disintegrant is in a range from 2.0% and 4.0% by weight with respect to the total weight of the orally dispersible tablet and the amount of oxazepam is in a range from 20.0% and 30.0% by weight with respect to the total weight of the orally dispersible tablet.

Another embodiment of the second aspect of the present invention is directed to the preparation of an orally dispersible tablet as defined in the first aspect comprising:
i) providing oxazepam or a pharmaceutically acceptable salt thereof;
ii) (a) blending said oxazepam or pharmaceutically acceptable salt thereof with a mixture comprising a diluent, selected from the group consisting of lactose monohydrate, microcrystalline cellulose, cellulose powder and silicified microcrystalline cellulose, and a disintegrant selected from the group consisting of sodium croscarmellose, crospovidone, low-substituted hydroxypropyl cellulose and mixtures thereof,
   (b) granulating the resulting blend obtained in step (a);
iii) screening the granules resulting from step (ii)(b);
iv) blending the screened granules of step (iii) with an extragranular mixture comprising a disintegrant selected from the group consisting of sodium croscarmellose, crospovidone, low-substituted hydroxypropyl cellulose and mixtures thereof, and
v) compressing the blend resulting from step (vi) to provide a tablet,
wherein the amount of disintegrant is in a range from 6.0% and 10.0% by weight with respect to the total weight of the orally dispersible tablet and the amount of oxazepam is in a range from 6.0 and 30% by weight with respect to the total weight of the orally dispersible tablet.

In another more preferred embodiment of the second aspect, the disintegrant is croscarmellose sodium.

An orally dispersible tablet obtained by the process of the second aspect of the present invention is also part of the invention.

Also, the orally dispersible tablets according to the first aspect, for use in the treatment of anxiety, insomnia and in the control of symptoms of alcohol withdrawal syndrome is part of the present invention.

### Examples

Below we will illustrate the invention by means of orally dispersible tablets prepared by the inventors, which demonstrate their disintegration time as well as their dissolution profile.

### Comparative Example A

This example was prepared by a method of direct compression, as explained herein: Crospovidone (53 g) and calcium silicate (160 g) were sieved and blended together. Lactose monohydrate (930 g), low-substituted hydroxypropyl cellulose (71 g) (and oxazepam (556 g) were sieved and blended together. Both blends were blended together.

Colloidal anhydrous silica (9 g) was sieved and blended with the previous blend. Magnesium stearate (18 g) was sieved and blended with the blend resulting from the previous step. The final blend is compressed to provide a tablet.

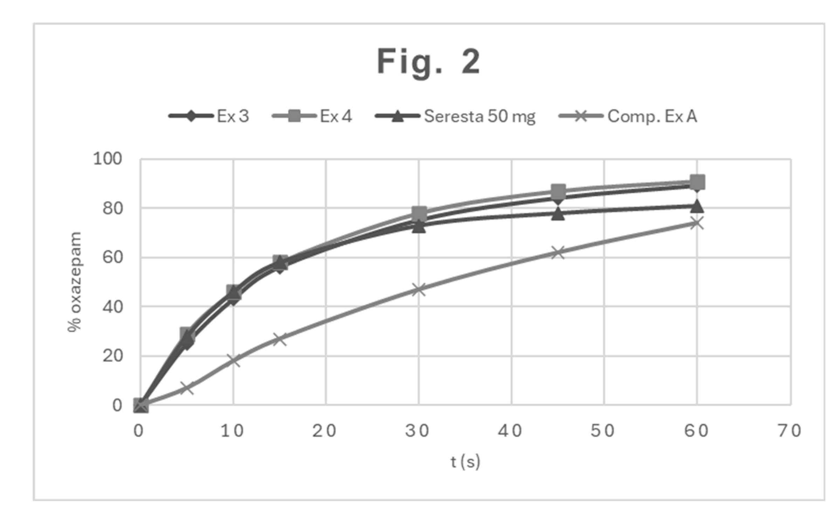

As shown in Figure 2, the dissolution profile of this example was not comparable to that of Seresta^{®} 50 mg.

### Example 1

Oxazepam (317 g), lactose monohydrate (3745 g), low-substituted hydroxypropyl cellulose (210 g), povidone (131 g) and crospovidone (157 g) were blended during 10 minutes in a high-shear mixer granulator to obtain a blend. The blend was granulated in the high-shear mixer granulator using water as granulation fluid. The granulate was dried in a fluid bed dryer and the dried granulate was screened. Calcium silicate (472 g), crospovidone (157 g) and colloidal anhydrous silica (26 g) were sieved and blended with the screened granules for 10 minutes.

Magnesium stearate (34 g) was sieved and blended with the blend obtained above for 5 minutes.

The resulting blend was compressed to provide a tablet.

### Example 2

Oxazepam (437 g), lactose monohydrate (2950 g), microcrystalline cellulose (805 g) and rice starch (597 g) were blended during 10 minutes in a high-shear mixer granulator to obtain a first blend.

The granulating solution was prepared by dispersing rice starch in a part of purified water, heating another part of purified water at around 97°C and adding it to the starch dispersion to form a paste.

The first blend was granulated in the high-shear mixer granulator using starch paste and the wet granulate is screened. The screened granulate was dried in a fluid bed dryer and the resulting dried mixture was subsequently screened.

Rice starch (146 g), sodium croscarmellose (157 g), sodium saccharin (26 g) and talc (87 g) were sieved and blended with the screened dried granules previously obtained for 15 minutes.

The resulting blend was blended with sieved magnesium stearate (44 g) for 7 minutes,
Finally, the resulting blend was compressed to obtain a tablet.

### Example 3

Oxazepam (1406 g), lactose monohydrate (2420 g), microcrystalline cellulose (665 g) and rice starch (660 g) were blended during 10 minutes in a high-shear mixer granulator to obtain a blend.

The granulating solution was prepared by dispersing rice starch in a part of purified water, heating another part of purified water at around 97°C and adding it to the starch dispersion to form a paste.

The blend obtained above was granulated in the high-shear mixer granulator using the starch paste, screened and dried in a fluid bed dryer. The resulting dried granulate was subsequently screened to obtain screened granules.

Microcrystalline cellulose (140 g), sodium croscarmellose (170 g), sodium saccharin (28 g) and talc (94 g) were sieved and blended with the screened granules previously obtained for 15 minutes and the resulting mixture was blended for 7 minutes with sieved magnesium stearate (47 g).

The resulting blend was compressed to provide a tablet.

### Example 4

Oxazepam (500 g), lactose monohydrate (820 g), low-substituted hydroxypropyl cellulose (66 g), crospovidone (29 g) and povidone (33 g) were blended during 10 minutes in a high-shear mixer granulator. The blend was granulated in the high shear mixer granulator using purified water and dried in a fluid bed dryer. The resulting dried granulate was screened and blended together with a sieved mixture of calcium silicate (150 g) and crospovidone (29 g) for 10 minutes. The resulting blend was blended with sieved colloidal anhydrous silica (8 g) for 10 minutes and the resulting blend was further blended for 5 minutes with sieved magnesium stearate (16 g). Finally, the resulting blend was compressed to provide a tablet.

### Disintegration time and mechanical properties

The disintegration time of each of the examples was measured according to the standard test A described in the European Pharmacopeia. 2.9.1.

As shown in Table 1 below, all the disintegration times were below 30 seconds, which was the time considered adequate for an orally dispersible.

On the other hand, the orally dispersible tablets of the examples showed excellent mechanical properties in terms of resistance to crushing and friability.

The mean resistance to crushing and the friability were measured after 3 months at 40°C and 75% relative humidity in a blister packaging of Aluminium-Aluminium (Alu-Al)

**Table 1. Disintegration time and mechanical properties of the orally dispersible tablets of examples 1-4.**

| | **Example 1** | **Example 2** | **Example 3** | **Example 4** |
|---|---|---|---|---|
| Disintegration time (s) | 27 | 20 | 18 | 29 |
| Mean resistance to crushing (N) | 31 | 19 | 55 | - |
| Friability (%) | -- | 0.1 | 0.2 | -- |

### Dissolution profile

The dissolution profile of each of the examples was measured using dissolution equipment, which meets the specifications described in the European Pharmacopoeia, equipped with paddle apparatus as stirring system. The tests were performed in the following conditions:

| | |
|---|---|
| Apparatus: | Paddle apparatus (Ph. Eur.) |
| Stirring rate: | 50 r.p.m. |
| Dissolution medium: | Hydrochloric acid 0.1 N (Ph. Eur.) |
| Volume: | 900 ml |
| Sampling times: | 5, 10, 15, 30, 45 and 60 minutes. |

Table 2 shows the percentage of oxazepam dissolved over time from orally dispersible tablets of examples 1 and 2.

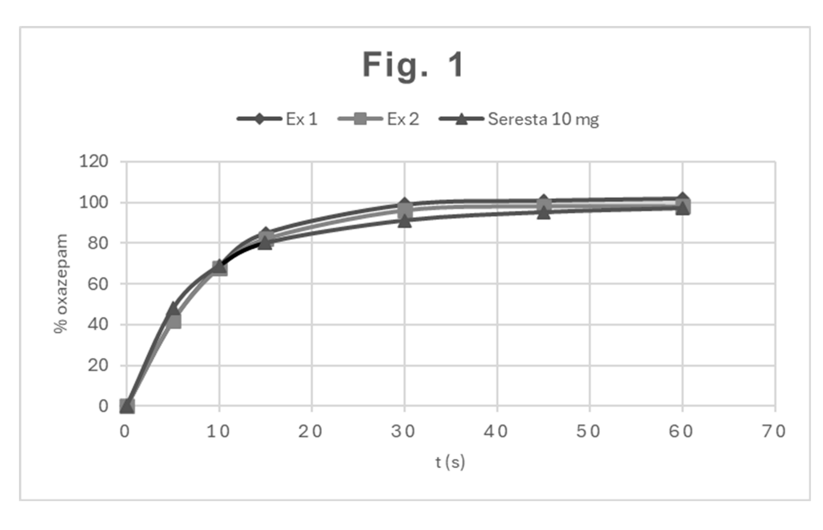

As shown in Table 2 and Figure 1, examples 1 and 2 showed a dissolution profile with a similar pattern as the 10 mg strength of Seresta^{®}. This table is also represented in Fig 1.

**Table 2. Dissolution profile of Examples 1 and 2.**

| **Time (min)** | **Example 1** | **Example 2** | **Seresta^{®} 10 mg** |
|---|---|---|---|
| 5 | 42 | 42 | 48 |
| 10 | 69 | 68 | 69 |
| 15 | 85 | 82 | 80 |
| 30 | 99 | 96 | 91 |
| 45 | 101 | 98 | 95 |
| 60 | 102 | 98 | 97 |

As shown in Table 3 and Fig. 2, the dissolution profiles of comparable example A, which was prepared by direct compression as explained above, was not adequate as it was not comparable to that of Seresta^{®}. However, examples 3 and 4 which were prepared according to the second aspect of the present invention showed a dissolution profile comparable to the 50 mg strength of Seresta^{®}.

This table is also represented in Fig 2.

**Table 3. Dissolution profile of Comparative examples A, examples 3 and 4.**

| **Time (min)** | **Comparative example A** | **Example 3** | **Example 4** | **Seresta^{®} 50 mg** |
|---|---|---|---|---|
| 5 | 7 | 25 | 29 | 28 |
| 10 | 18 | 43 | 46 | 46 |
| 15 | 27 | 56 | 58 | 58 |
| 30 | 47 | 75 | 78 | 73 |
| 45 | 62 | 84 | 87 | 78 |
| 60 | 74 | 89 | 91 | 81 |

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

## Claims

1. An orally disintegrating tablet comprising an intragranular and an extragranular phase, comprising:
a) oxazepam or a pharmaceutically acceptable salt thereof, and
b) one or more disintegrants in an amount from 0.5 to 12.0% by weight with respect to the total weight of the orally disintegrating tablet , and selected from the group consisting of sodium croscarmellose, crospovidone, low-substituted hydroxypropyl cellulose and mixtures thereof,
wherein the oxazepam or the pharmaceutically acceptable salt thereof is in the intragranular phase.

2. The orally dispersible tablet according to claim 1, wherein the one or more disintegrants are either in the extragranular phase or in both the extragranular and the intragranular phase.

3. The orally dispersible tablet according to any of the claims 1-2, wherein the amount of disintegrant is in a range from 1.0 to 8.0% by weight.

4. The orally dispersible tablet according to any of the claims 1-3, wherein the disintegrant is croscarmellose sodium.

5. The orally dispersible tablet according to any of the claims 1-4, wherein the amount of oxazepam is in a range from 5.0% to 35.0% by weight with respect to the total weight of the orally dispersible tablet.

6. The orally dispersible tablet according to any of the claims 1-5, further comprising one or more lubricants, selected from the group consisting of calcium stearate, zinc stearate, glyceryl behenate, magnesium stearate and mineral oil, preferably magnesium stearate.

7. The orally dispersible tablet according to claim 6, wherein the amount of lubricant is in a range from 0.6% to 1.0% by weight with respect to the total weight of the orally dispersible tablet.

8. The orally dispersible tablet according to any of the claims 1-7, wherein the disintegration time of the tablet is in a range from 10 to 30 seconds, measured according to the standard test A described in the European Pharmacopeia. 2.9.1.

9. A process for the preparation of an orally dispersible tablet as defined in any of the claims 1-8 comprising:
i) providing oxazepam or a pharmaceutically acceptable salt thereof;
ii) (a) blending said oxazepam or pharmaceutically acceptable salt thereof with a mixture comprising a diluent, selected from the group consisting of lactose monohydrate, microcrystalline cellulose, cellulose powder and silicified microcrystalline cellulose, and optionally a disintegrant, selected from the group consisting of sodium croscarmellose, crospovidone, low-substituted hydroxypropyl cellulose and mixtures thereof,
(b) granulating the resulting blend obtained in step (a);
iii) screening the granules resulting from step (ii)(b);
iv) blending the screened granules of step (iii) with an extragranular mixture of excipients optionally comprising a disintegrant, and
v) compressing the blend resulting from step (vi) to provide a tablet,
wherein the amount of disintegrant is in a range from 0.5% to 12.0% by weight with respect to the total weight of the orally dispersible tablet.

10. The process according to claim 9, wherein the blend of step (ii)(a) does not comprise a disintegrant.

11. The process according to any of the claims 9-10, wherein the extragranular excipients comprise a disintegrant.

12. The process according to any of the claims 9-11, where the granulation of step (ii)(b) is a wet granulation with an aqueous solution comprising a binder selected from the group consisting of povidone, rice starch, gelatine and polyethylene glycol, preferably rice starch; and has the further step (c) of drying in a fluid bed dryer the granules resulting from step (ii)(b).

13. The process according to any of the claims 9-12, wherein the extragranular excipients comprises a lubricant selected from the group consisting of calcium stearate, zinc stearate, glyceryl behenate, magnesium stearate and mineral oil, preferably magnesium stearate.

14. An orally dispersible tablet obtainable by the process of any of the claims 9-13.

15. An orally dispersible tablet according to any of the claims 1-8 for use in the treatment of anxiety, insomnia and in the control of symptoms of alcohol withdrawal syndrome.
